# EUROPÄISCHE PATENTSCHRIFT

(11) **EP 1 915 958 B1**
(45) Veröffentlichungstag und Bekanntmachung des Hinweises auf die Patenterteilung: **11.08.2010**
(21) Anmeldenummer: 07118745.4
(22) Anmeldetag: 18.10.2007
(51) Int. Cl.: A61B 17/70, A61B 17/80

(54) **Implantat und Implantatsystem**
Implant and implant system
Implant et système d'implant

(30) Priorität: 24.10.2006 US 585760; 10.11.2006 DE 102006053880
(43) Veröffentlichungstag der Anmeldung: 30.04.2008
(73) Patentinhaber: Aesculap AG, 78532 Tuttlingen (DE)
(72) Erfinder: Beger, Jens, 78532 Tuttlingen (DE); Peukert, Andrea, 78532 Tuttlingen (DE); Schumacher, Jörg, 78532 Tuttlingen (DE)
(74) Vertreter: Hoeger, Stellrecht & Partner Patentanwälte

(56) Entgegenhaltungen:
- EP-A- 1 348 390
- FR-A- 2 651 992
- FR-A- 2 784 571
- US-A- 5 531 747
- US-A1- 2004 127 904

## Beschreibung

Die vorliegende Erfindung betrifft ein Implantat zum Verbinden zweier Wirbelkörper einer menschlichen oder tierischen Wirbelsäule umfassend einen Implantatgrundkörper und mindestens ein Befestigungselementhalteglied, an welchem mindestens ein Teil eines mindestens teilweise in einen Wirbelkörper einbringbaren Befestigungselements anorden-, lager- und/oder festlegbar ist wobei das mindestens eine Befestigungselementhalteglied und der Implantatgrundkörper miteinander lösbar verbindbar sind, wobei in einer Montagestellung das mindestens eine Befestigungselementhalteglied und der Implantatgrundkörper voneinander getrennt und in einer Verbindungsstellung miteinander verbunden sind, wobei eine Verbindungsvorrichtung vorgesehen ist zum Verbinden des Implantatgrundkörpers und des mindestens einen Befestigungselementhalteglieds in der Verbindungsstellung, dass die Verbindungsvorrichtung mindestens ein erstes und mindestens ein zweites, mit dem mindestens einen ersten Verbindungsglied in Eingriff bringbares Verbindungsglied umfasst, dass das mindestens eine erste und das mindestens eine zweite Verbindungsglied in der Verbindungsstellung in Eingriff stehen, wobei das mindestens eine erste Verbindungsglied am Implantatgrundkörper und das mindestens eine zweite Verbindungsglied am mindestens einen Befestigungselementhalteglied angeordnet ist.

Ferner wird nachfolgend ein Implantatsystem beschrieben zum Verbinden zweier Wirbelkörper einer menschlichen oder tierischen Wirbelsäule mit mindestens zwei mindestens teilweise in einen Wirbelkörper einbringbaren Befestigungselementen und einem Implantat umfassend einen Implantatgrundkörper und mindestens ein Befestigungselementhalteglied, an welchem mindestens einen Teil eines der Befestigungselemente anorden-, lager- und/oder festlegbar ist.

Es ist bekannt, in der Wirbelsäulenchirurgie Fusionen von instabilen Segmenten der Wirbelsäule mittels Implantaten und Implantatsystemen der eingangs beschriebenen Art vorzunehmen. Derartige Implantatsysteme, die auch als Fixateure bezeichnet werden, werden über dorsale und/oder ventrale Zugänge in den menschlichen oder tierischen Körper eingebracht. Es sind Implantatgrundkörper mit mindestens einem Befestigungselementhalteglied, insbesondere in Form einer Durchbrechung, durch welche beispielsweise ein Befestigungselement in Form einer Knochenschraube durchgesteckt und so der Implantatgrundkörper an einem der zwei miteinander zu verbindenden Wirbelkörper festgelegt werden kann, bekannt. Allerdings haben diese Implantate den Nachteil, dass zuerst der Implantatgrundkörper in seine gewünschte Position gebracht werden muss und erst dann das Befestigungselement in das Befestigungselementhalteglied eingeführt und mit dem Wirbelkörper verschraubt werden kann. Alternativ wäre es allenfalls denkbar, das Befestigungselement vor Einführen des Implantatgrundkörpers am Befestigungselementhalteglied zu lagern und somit die Einheit aus Implantatgrundkörper und Befestigungselement zusammen in den Körper einzusetzen. Nachteilig dabei ist jedoch, dass ein Großteil des Operationsfeldes verdeckt wird. Insbesondere können die Befestigungselemente bei dieser Vorgehensweise nur sehr unpräzise in den jeweiligen Wirbelkörper eingebracht werden.

Aus der FR 2 651 992 A1 ist ein Osteosyntheseimplantat für die Wirbelsäule bekannt. Ein System zur Stabilisierung der Wirbelsäule ist in der US 5,531,747 beschrieben. Ein Knochenplattensystem ist in der EP 1 348 390 A2 offenbart. Und schließlich ist aus der FR 2 784 571 A1 eine Osteosyntheseplatte zum Verbinden von lumbalen Wirbelkörpern bekannt.

Es ist daher Aufgabe der vorliegenden Erfindung, ein Implantat der eingangs beschriebenen Art so zu verbessern, dass mit ihm zwei Wirbelkörper auf einfache Weise verbunden werden können.

Diese Aufgabe wird bei einem Implantat der eingangs beschriebenen Art erfindungsgemäß dadurch gelöst, dass die Verbindungsvorrichtung in Form einer Rast- oder Schnappverbindung mit mindestens einem ersten Rastglied und mindestens einem zweiten Rastglied ausgebildet ist und dass das mindestens eine Befestigungselementhalteglied in der Verbindungsstellung relativ zum Implantatgrundkörper bewegbar ist.

Durch die erfindungsgemäß vorgeschlagene, mögliche Trennung zwischen dem Implantatgrundkörper und dem mindestens einen Befestigungselementhalteglied wird es möglich, beispielsweise zuerst die Befestigungselemente mindestens teilweise in die miteinander zu verbindenden Wirbelkörper einzubringen, dann zum Beispiel die beiden Wirbelkörper über eine entsprechende Spreizvorrichtung, die an den Befestigungselementen angreifen kann, in eine gewünschte Relativposition zu distrahieren, daran anschließend, falls das mindestens eine Befestigungselementhalteglied nicht bereits an einem Befestigungselement vormontiert ist, das mindestens eine Befestigungselementhalteglied am eingebrachten Befestigungselement anzuordnen, zu lagern und/oder festzulegen, und in einem abschließenden Schritt den Implantatgrundkörper einzubringen und mit dem mindestens einen Befestigungselementhalteglied zu verbinden. Die Befestigungselemente können insbesondere als Führungen für Einsetzinstrumente und/oder den Implantatgrundkörper dienen. In jedem Fall muss der Implantatgrundkörper nicht als erstes an die Wirbelkörper angelegt werden, so dass er auch das Operationsfeld nicht verdeckt, insbesondere dann, wenn die Befestigungselemente in die Wirbelkörper eingebracht werden. Vorteilhaft ist es, dass das mindestens eine Befestigungselementhalteglied in der Verbindungsstellung relativ zum Implantatgrundkörper bewegbar ist. Dies ermöglicht es, den Implantatgrundkörper noch nach dem Verbinden mit dem mindestens einen Befestigungselementhalteglied in eine endgültige Position zu bewegen, allerdings mit dem Vorteil, dass das mindestens eine Befestigungselementhalteglied bereits am Implantatkörper vorfixiert ist. Vorteilhaft ist es, dass eine Verbindungsvorrichtung vorgesehen ist zum Verbinden des Implantatgrundkörpers und des mindestens einen Befestigungselementhalteglieds in der Verbindungsstellung, wenn die Verbindungsvorrichtung mindestens ein erstes und mindestens ein zweites, mit dem mindestens einen ersten Verbindungsglied in Eingriff bringbares Verbindungsglied umfasst, wenn das mindestens eine erste und das mindestens eine zweite Verbindungsglied in der Verbindungsstellung in Eingriff stehen, wenn das mindestens eine erste Verbindungsglied am Implantatgrundkörper und wenn das mindestens eine zweite Verbindungsglied am mindestens einen Befestigungselementhalteglied angeordnet ist. Die somit mindestens zweiteilige Verbindungsvorrichtung ermöglicht eine Verbindung zwischen dem Implantatgrundkörper und dem mindestens einen Befestigungselementhalteglied auf einfache Weise. Eine Verbindung setzt lediglich voraus, dass das mindestens eine erste und das mindestens eine zweite Verbindungsglied in Eingriff gebracht werden. Besonders einfach wird der Aufbau des Implantats dadurch, dass die Verbindungsvorrichtung in Form einer Rast- oder Schnappverbindung mit mindestens einem ersten Rastglied und mindestens einem zweiten Rastglied ausgebildet ist. Eine Verbindung zwischen dem Implantatgrundkörper und dem mindestens einen Verbindungselementhalteglied kann durch eine Rast- oder Schnappverbindung ohne zusätzliche Werkzeuge oder Instrumente hergestellt werden, beispielsweise durch Verrasten oder Aufklicken des einen Teils des Implantats auf den zugeordneten anderen Teil.

Grundsätzlich wäre es denkbar, dass nur ein mit dem Implantatkörper lösbar verbindbares Verbindungselementhalteglied vorgesehen ist. Ein Befestigungselement kann dann beispielsweise an einem unlösbar mit dem Implantatgrundkörper, insbesondere einstückig mit diesem ausgebildeten Befestigungselementhalteglied gelagert sein. Es könnte dann der Implantatkörper mit einem ersten, somit direkt am Implantatgrundkörper gelagerten Befestigungselement an einem der beiden Wirbelkörper vorfixiert werden, und das weitere Befestigungselementhalteglied könnte dann mit einem in den anderen Wirbelkörper eingebrachten Befestigungselement verbunden werden. Besonders vorteilhaft ist es jedoch, wenn zwei mit dem Implantatgrundkörper lösbar verbindbare Befestigungselementhalteglieder vorgesehen sind. Beispielsweise dann, wenn zwei Befestigungselemente zum Festlegen des Implantatgrundkörpers an den beiden zu verbindenden Wirbelkörpern vorgesehen sind, können zunächst diese in die beiden Wirbelkörper eingetrieben werden und erst nach ihrer endgültigen Positionierung kann dann der Implantatgrundkörper mit den beiden Befestigungselementhaltegliedern verbunden werden. Selbstverständlich kann das Implantat auch derart ausgebildet sein, dass eine der Zahl der benötigten Befestigungselemente entsprechende Anzahl von mit dem Implantatgrundkörper lösbar verbindbaren Befestigungselementhaltegliedern vorgesehen ist.

Es wäre zwar möglich, das mindestens eine Befestigungselementhalteglied in der Verbindungsstellung relativ zum Implantatkörper verdrehbar oder verschwenkbar anzuordnen, vorzugsweise ist das mindestens eine Befestigungselementhalteglied in der Verbindungsstellung jedoch relativ zum Implantatgrundkörper verschiebbar. Durch eine solche verschiebbare Lagerung der beiden Teile aneinander in der Verbindungsstellung kann auf einfache und definierte Weise eine gewünschte Relativposition zwischen dem mindestens einen Befestigungselementhalteglied und dem Implantatgrundkörper eingestellt werden.

Günstig ist es, wenn das mindestens eine Befestigungselementhalteglied in der Verbindungsstellung am Implantatgrundkörper unbeweglich gehalten ist. Auf diese Weise kann sofort durch das Verbinden des mindestens einen Befestigungselementhalteglieds mit dem Implantatgrundkörper eine definierte Position der beiden Teile relativ zueinander erreicht werden. Sind beispielsweise zwei oder mehr Befestigungselementhalteglieder vorgesehen, so kann der Implantatgrundkörper zunächst mit einem Befestigungselementhalteglied verbunden werden, so dass diese in der Verbindungsstellung relativ zueinander unbeweglich sind. Dies gestattet es, das weitere oder die weiteren Befestigungselementhalteglieder zunächst, falls erforderlich, in eine Position zu bringen, in der sie mit dem Implantatgrundkörper verbindbar sind. Ferner kann so noch eine Nachjustierung erfolgen, wenn die weiteren Befestigungselementhalteglieder relativ zum Implantatgrundkörper bewegbar sind.

Günstig ist es, wenn das mindestens eine erste Rastglied in Form einer Rastausnehmung ausgebildet ist und wenn das mindestens eine zweite Rastglied in Form eines federnd gelagerten Rastvorsprungs ausgebildet ist. Eine solche Ausgestaltung der Rastglieder macht es möglich, die miteinander zu verbindenden Teile des Implantats in die gewünschte Relativposition zu bringen, wobei dann eine Verbindung automatisch dadurch hergestellt wird, dass der federnd gelagerte Rastvorsprung in die korrespondierende Rasteinnehmung ganz oder teilweise eintaucht.

Um die Stabilität des Implantats zu verbessern und zusätzlich zu vermeiden, dass Teile desselben in unerwünschter Weise vorstehen, ist es vorteilhaft, wenn am Implantatgrundkörper eine Befestigungselementhaltegliedaufnahme ausgebildet ist, in welche(r) das mindestens eine Befestigungselementhalteglied in der Verbindungsstellung mindestens teilweise eintaucht und/oder gelagert ist. Zudem ist das Befestigungselementhalteglied in der Befestigungselementhaltegliedaufnahme auch mindestens teilweise geschützt. Dadurch kann insbesondere eine zum Beispiel zusätzlich vorgesehene Verbindungsvorrichtung gegen äußere Einflüsse, die ein Lösen der Verbindung bewirken könnten, geschützt werden.

Eine besonders sichere Verbindung zwischen dem Implantatgrundkörper und dem mindestens einen Befestigungselementhalteglied kann dadurch erreicht werden, dass das mindestens eine erste oder das mindestens eine zweite Verbindungsglied der Verbindungsvorrichtung an der Befestigungselementhaltegliedaufnahme angeordnet ist. Insbesondere dann, wenn das an der Befestigungselementhaltegliedaufnahme angeordnete mindestens eine Verbindungsglied in einer Ausnehmung oder an einem Rücksprung angeordnet ist, wird ein unerwünschtes und unbeabsichtigtes Lösen der Verbindung zwischen dem Implantatgrundkörper und dem mindestens einen Befestigungselementhalteglied in der Verbindungsstellung deutlich erschwert oder sogar unmöglich.

Auf besonders einfache Weise lassen sich der Implantatgrundkörper und das mindestens eine Verbindungselementhalteglied miteinander verbinden, wenn das mindestens eine erste Verbindungsglied in Form einer hinterschnittenen Verbindungsgliedaufnahme und wenn das mindestens eine zweite Verbindungsglied in Form eines zur Verbindungsgliedaufnahme im Querschnitt korrespondierenden Verbindungsabschnitts ausgebildet ist, welcher in der Verbindungsstellung von der Verbindungsgliedaufnahme in einem gesamten Winkelbereich von mehr als 180° umgriffen wird. Beispielsweise kann der Verbindungsabschnitt ein stabförmiger Abschnitt des Implantatgrundkörpers sein, insbesondere auch ein stabförmiger Abschnitt an einem im Wesentlichen plattenförmigen Implantatgrundkörper. Selbstverständlich könnte der Verbindungsabschnitt auch an einer rein stabförmigen Struktur vorgesehen sein, was es grundsätzlich ermöglichen würde, das mindestens eine Verbindungselementhalteglied in verschiedenen Relativpositionen am Implantatgrundkörper anzuordnen und mit diesem zu verbinden. Ferner lässt sich so beispielsweise der Teil des Implantats, an dem die Verbindungsgliedaufnahme angeordnet ist, auf einfache Weise auf den Verbindungsabschnitt aufclipsen, beispielsweise dann, wenn die Verbindungsgliedaufnahme in Form einer Nut ausgebildet ist, die jedoch mindestens eine, vorzugsweise zwei Hinterschneidungen aufweist, so dass sie den Verbindungsabschnitt auf mehr als 180° umgreift.

Besonders einfach in der Herstellung wird das Implantat, wenn der Verbindungsabschnitt stabförmig und im Querschnitt rund, oval, rechteckig, unrund oder im Wesentlichen rund, oval, rechteckig oder unrund geformt ist.

Die Stabilität des Implantats lässt sich insbesondere dadurch erhöhen, dass zwei erste und zwei zweite Verbindungsglieder vorgesehen sind und dass die zwei ersten und/oder die zwei zweiten Verbindungsglieder parallel oder im Wesentlichen parallel zueinander ausgerichtet sind. Ferner erlaubt eine derartige Ausgestaltung auch, die Verbindungsglieder so auszubilden, dass auf einfache Weise eine Relativbewegung zwischen dem Implantatgrundkörper und dem mindestens einen Befestigungselementhalteglied möglich ist, insbesondere in einer Richtung parallel zu einer von den Verbindungsgliedern vorgegebenen Richtung.

Gemäß einer bevorzugten Ausführungsform der Erfindung kann vorgesehen sein, dass der Implantatgrundkörper mindestens eine Einführdurchbrechung aufweist, die derart bemessen ist, dass das mindestens eine Befestigungselementhalteglied nur in einer Einführstellung oder in der Montagestellung durch die mindestens eine Einführdurchbrechung einführbar ist, in welcher Längsachsen des Implantatgrundkörpers und des mindestens einen Befestigungselementhalteglieds relativ zueinander in einem vorgegebenen Winkelbereich ausgerichtet sind. So kann insbesondere der Implantatgrundkörper auf einfache Weise mittels des mindestens einen Befestigungselementhalteglieds beispielsweise an einem bereits in einem der miteinander zu verbindenden Wirbelkörper eingebrachten Befestigungselement gehalten werden. Dies kann insbesondere dadurch geschehen, dass das mindestens eine Befestigungselementhalteglied relativ zum Implantatgrundkörper aus der Einführ- oder Montagestellung heraus bewegt wird. Mit anderen Worten ist es hierfür also nur erforderlich, dass eine relative Einstellung der Längsachsen des Implantatgrundkörpers und des mindestens einen Befestigungselementhalteglieds nach dem Einführen verändert wird.

Besonders einfach wird der Aufbau des Implantats, wenn die Einführdurchbrechung langgestreckt oder in Form eines Langlochs ausgebildet ist. Beispielsweise kann so ein im Wesentlichen quaderförmiges Befestigungselementhalteglied durch die Einführdurchbrechung dann durchgesteckt werden, wenn deren Längsachsen parallel oder im Wesentlichen parallel zueinander ausgerichtet sind. Ein unbeabsichtigtes Lösen des Implantatgrundkörpers von insbesondere einem Befestigungselementhalteglied, welches an einem bereits in einen Wirbelkörper eingebrachten Befestigungselement gehalten oder gelagert ist, kann durch einfaches Verdrehen des Befestigungselementhalteglieds relativ zum Implantatgrundkörper geschehen, beispielsweise um eine durch das Befestigungselement vorgegebene Längsachse.

Um ein Befestigungselement auf einfache Weise an dem mindestens einen Befestigungselementhalteglied anordnen, lagern oder festlegen zu können, ist es vorteilhaft, wenn das mindestens eine Befestigungselementhalteglied eine Befestigungselementaufnahme für ein Befestigungselement aufweist.

Gemäß einer weiteren bevorzugten Ausführungsform der Erfindung kann ferner vorgesehen sein, dass mindestens zwei Befestigungselementhalteglieder vorgesehen sind und dass mindestens zwei der mindestens zwei Befestigungselementhalteglieder Befestigungselementaufnahmen aufweisen, deren Längsachsen in der Verbindungsstellung parallel oder im Wesentlichen parallel zueinander ausgerichtet sind. Es ist so möglich, Befestigungselemente parallel oder im Wesentlichen parallel in die miteinander zu verbindenden Wirbelkörper einzubringen. Dadurch können insbesondere Einsetzinstrumente zum Einbringen der Befestigungselemente in die Wirbelkörper als Einführhilfen für den Implantatgrundkörper genutzt werden. Ferner wird so vermieden, dass sich beispielsweise sehr lange Befestigungselemente gegenseitig behindern, beispielsweise wenn diese in denselben Wirbelkörper eingebracht werden.

Besonders einfach und sicher lassen sich Befestigungselemente am mindestens einen Befestigungselementhalteglied anordnen, lagern oder festlegen, wenn die Befestigungselementaufnahme in Form einer Durchbrechung ausgebildet ist. Ein Befestigungselement kann durch die Durchbrechung beispielsweise durchgesteckt oder, wenn diese mit einem Gewinde oder einer andersartigen Verbindungsvorrichtung ausgestattet ist, verschraubt oder anderweitig verbunden werden.

Vorteilhaft wäre es, wenn das mindestens eine Befestigungselementhalteglied relativ zu einem Befestigungselement, an welchem es gelagert, angeordnet oder festgelegt ist, bewegbar wäre. Hierfür ist es günstig, wenn die Befestigungselementaufnahme einen Lagersitz für einen Gelenkkopf eines Befestigungselements umfasst. Insbesondere kann so das Befestigungselementhalteglied relativ zum Befestigungselement um ein Zentrum des Lagersitzes in nahezu beliebiger Weise verschwenkt werden.

Um eine Stabilität des Implantats zu erhöhen und/oder eine relative Bewegbarkeit des Befestigungselementhalteglieds relativ zu einem darin angeordneten oder gelagerten Befestigungselement zu begrenzen, ist es günstig, wenn sich an die Befestigungselementaufnahme mindestens ein Führungsabschnitt anschließt.

Besonders einfach wird der Aufbau des Implantats, wenn der mindestens eine Führungsabschnitt hülsenförmig oder im Wesentlichen hülsenförmig ausgebildet ist. Er kann insbesondere zur Stabilisierung einer Relativposition des Befestigungselementhalteglieds und eines Befestigungselements dienen, beispielsweise wenn Letzteres in Form einer Knochenschraube oder eines Knochennagels ausgebildet ist.

Vorteilhafterweise weisen das mindestens eine Befestigungselementhalteglied und/oder der Implantatgrundkörper mindestens ein Knochen- oder Gewebeverankerungselement auf. Mit Letzterem wird insbesondere eine Relativbewegung des Implantatkörpers und/oder des Befestigungselementhalteglieds relativ zu den Knochen- oder Gewebeteilen verhindert, an denen sie anliegen.

Besonders einfach und effizient lässt sich ein Knochen- oder Gewebeverankerungselement ausgestalten, wenn es in Form eines abstehenden Dorns ausgebildet ist. Das mindestens eine Knochen- oder Gewebeverankerungselement kann so ganz oder teilweise in Knochen oder Gewebe eindringen und eine unerwünschte Relativbewegung des Implantatgrundkörpers oder des Befestigungselementhalteglieds verhindern oder minimieren. Insbesondere kann ein solches Knochen- oder Gewebeverankerungselement als Verdrehsicherung dienen.

Um das Implantat optimal an relativ zueinander geneigte Flächen von miteinander zu verbindenden Wirbelkörpern anlegen zu können, ist es vorteilhaft, wenn der Implantatgrundkörper eine äußere Wirbelkörperanlagefläche aufweist, wenn die Wirbelkörperanlagefläche mindestens zwei Flächenabschnitte aufweist und wenn die Flächenabschnitte relativ zueinander um einen Anlagewinkel geneigt sind. Besonders vorteilhaft ist eine derartige Ausgestaltung zum Verbinden instabiler Segmente der Wirbelsäule im lumbosakralen Bereich, insbesondere bei Instabilitäten zwischen dem 5. Lendenwirbel und dem Kreuzbein (Os sacrum).

Vorteilhafterweise weist der Anlagewinkel einen Wert in einem Bereich von 20° bis 80° auf. Vorzugsweise beträgt der Wert 40° bis 60°. Derartige Anlagewinkel sind optimal, um praktisch beliebige Wirbel der Wirbelsäule miteinander zu verbinden.

Um den Implantatgrundkörper in gewünschter Weise an zwei miteinander zu verbindende Wirbelkörper individuell anpassen zu können, ist es vorteilhaft, wenn der Implantatgrundkörper einen ersten und einen mit dem ersten gelenkig verbundenen zweiten Teil umfasst und wenn der erste und der zweite Teil jeweils einen Flächenabschnitt definieren. Die beiden Flächenabschnitte können so in gewünschter Weise auch relativ zueinander ausgerichtet werden. Beispielsweise kann ein Gelenk aus einem Werkstoff ausgebildet sein, der sich von einem Werkstoff, aus dem der Implantatgrundkörper im Übrigen ausgebildet ist, unterscheidet. Denkbar sind insbesondere Film- oder Scharniergelenke, die einfach herzustellen sind und dennoch eine ausreichende Stabilität des Implantatgrundkörpers sicherstellen.

Besonders kompakt wird der Aufbau des Implantats, wenn die Wirbelkörperanlagefläche die mindestens eine Einführdurchbrechung mindestens teilweise begrenzt.

Damit eine Verbindung zwischen dem mindestens einen Befestigungselementhalteglied und dem Implantatgrundkörper auf einfache Weise visuell überprüft werden kann, ist es vorteilhaft, wenn die Wirbelkörperanlagefläche von der mindestens einen Befestigungselementhaltegliedaufnahme in entgegengesetzter Richtung weg weist.

Vorzugsweise weist das mindestens eine Befestigungselementhalteglied mindestens eine Werkzeugaufnahme für ein Halte- und/oder Einsetzinstrument auf. So kann verhindert werden, dass sich das Befestigungselementhalteglied in unerwünschter Weise relativ zum Implantatgrundkörper bewegen kann, beispielsweise beim Ineingriffbringen der beiden Teile miteinander.

Günstig ist es, wenn bei einem Implantatsystem der eingangs beschriebenen Art vorgesehen ist, dass das mindestens eine Befestigungselementhalteglied und der Implantatgrundkörper miteinander lösbar verbindbar sind, dass in einer Montagestellung das mindestens eine Befestigungselementhalteglied und der Implantatgrundkörper voneinander getrennt und in einer Verbindungsstellung miteinander verbunden sind. Es lassen sich so der Implantatgrundkörper und das mindestens eine Befestigungselementhalteglied auf einfache und sichere Weise verbinden, insbesondere nachdem die Teile relativ zueinander in eine gewünschte Stellung gebracht wurden. Selbstverständlich kann das Implantatsystem eine Mehrzahl von Implantaten umfassen, die auf unterschiedliche, miteinander zu verbindende Wirbelkörper abgestimmt sind. Analog können auch unterschiedliche Befestigungselementhalteglieder und Befestigungselemente vom Implantatsystem umfasst werden.

Vorteilhaft ist es, wenn das Implantat des Implantatsystems eines der oben beschriebenen Implantate ist. Ein solches Implantat zu verwenden hat die oben im Zusammenhang mit den erfindungsgemäß vorgeschlagenen Implantatweiterbildungen beschriebenen Vorteile.

Vorteilhaft kann es ferner sein, wenn mindestens eines der Befestigungselementhalteglieder in einer Einsetzstellung an einem der Befestigungselemente beweglich gelagert ist und wenn in einer Verbindungsstellung das mindestens eine Befestigungselementhalteglied unbeweglich an dem einen der Befestigungselemente festgelegt ist. Dadurch kann beispielsweise ein Befestigungselementhalteglied an einem Befestigungselement bereits nach der Herstellung vormontiert werden. Ein Zusammenbau im Operationssaal ist daher nicht mehr erforderlich, was den Zeitaufwand des Eingriffs deutlich reduziert. Ferner kann die bewegliche Lagerung in der Einführstellung den Einbau des Implantats in den menschlichen oder tierischen Körper vereinfachen, da eine endgültige Justierung beispielsweise erst nach dem Einbringen der Befestigungselemente in den oder die Wirbelkörper und nach Verbinden der Befestigungselementhalteglieder mit dem Implantatgrundkörper durchgeführt werden kann.

Vorzugsweise ist mindestens eines der Befestigungselemente in Form einer Knochenschraube ausgebildet. Es können prinzipiell alle herkömmlichen Knochenschrauben, die in der Wirbelsäulenchirurgie zum Einsatz kommen, verwendet werden.

Besonders vorteilhaft ist es jedoch, wenn die Knochenschraube in Form einer Polyaxialschraube ausgebildet ist, die einen Gelenkkopf umfasst und wenn mindestens eines der Befestigungselementhalteglieder eine zum Gelenkkopf korrespondierende Gelenkkopfaufnahme für den Gelenkkopf aufweist. Das mindestens eine Befestigungselementhalteglied und die Knochenschraube bilden quasi zusammen eine Polyaxialschraube, da es durch die Ausbildung des Gelenkkopfs an der eigentlichen Knochenschraube zusammen mit der Gelenkkopfaufnahme möglich ist, das Befestigungselementhalteglied relativ zur Knochenschraube, insbesondere zu einem Gewindeabschnitt derselben, zu verschwenken.

Damit eine gewünschte Relativposition zwischen dem mindestens einen Befestigungselement und dem mindestens einen Befestigungselementhalteglied dauerhaft eingestellt bleiben kann, ist es günstig, wenn mindestens eines der Befestigungselemente mit einem Fixierelement oder Sicherungsglied an dem mindestens einen Befestigungselementhalteglied festlegbar ist. Beispielsweise kann hierfür eine Mutter oder eine Fixierschraube dienen, mit der das Befestigungselementhalteglied und das Befestigungselement unbeweglich miteinander verbunden werden können.

Um die Befestigungselemente und die Implantatgrundkörper des Implantatsystems auf einfache Weise in einen menschlichen oder tierischen Körper einbringen zu können, ist es vorteilhaft, wenn das Implantatsystem ein Instrumentarium zum Einsetzen der Befestigungselemente in einen Wirbelkörper und/oder zum Festlegen mindestens eines Befestigungselements an mindestens einem Befestigungselementhalteglied umfasst.

Ferner wird nachfolgend auch ein Verfahren beschrieben zum Verbinden zweier Wirbelkörper eines menschlichen oder tierischen Körpers mit einem der oben beschriebenen Implantate oder mit einem der oben beschriebenen Implantatsysteme, umfassend:
- Eröffnen eines Zugangs zum menschlichen oder tierischen Körper,
- Einbringen jeweils mindestens eines Befestigungselementes in die beiden miteinander zu verbindenden Wirbelkörper, wobei mindestens an einem der Befestigungselemente ein Befestigungselementhalteglied gelagert ist,
- Ausrichten des mindestens einen Befestigungselementhaltegliedes relativ zu den Befestigungselementen derart, dass das mindestens eine Befestigungselementhalteglied durch eine am Implantatgrundkörper vorgesehene Einführöffnung einführbar ist,
- Einführen des Implantatgrundkörpers in den menschlichen Körper und Einführen des mindestens einen Befestigungselementhaltegliedes durch die Einführöffnung des Implantatgrundkörpers,
- Überführen der Befestigungselementhalteglieder relativ zum Implantatgrundkörper in die Verbindungsstellung und Verbinden der Befestigungselementhalteglieder mit dem Implantatgrundkörper.

Dieses Verfahren ist wesentlich einfacher und zeitsparender im Vergleich zu herkömmlichen Verfahren, da zum einen eine Vorfixierung der Befestigungselementhalteglieder und der Implantatgrundkörper aneinander ermöglicht wird und zum anderen eine instrumentenfreie oder im Wesentlichen instrumentenfreie Verbindung zwischen dem Implantatgrundkörper und dem mindestens einen Befestigungselementhalteglied und damit insbesondere auch indirekt zwischen dem Implantatgrundkörper und dem oder den in den Wirbelkörper oder sonstigen Knochen eingebrachten Befestigungselement oder Befestigungselementen.

Vorzugsweise wird ein ventraler Zugang zur Wirbelsäule eröffnet. Ein dorsaler Zugang ist für das beschriebene Verfahren nicht erforderlich, so dass zum einen nur ein Zugang benötigt wird, nämlich der ventrale Zugang, und zum anderen eben ganz auf den dorsalen Zugang verzichtet werden kann, welcher typischerweise mit einem deutlich höheren Verletzungsrisiko für das Rückenmark verbunden ist.

Die nachfolgende Beschreibung bevorzugter Ausführungsformen der Erfindung dient im Zusammenhang mit der Zeichnung der näheren Erläuterung. Es zeigen:
- Figur 1:: eine perspektivische Ansicht eines zwei Wirbelkörper miteinander verbindenden Ausführungsbeispiels eines erfindungsgemäßen Implantatsystems;
- Figur 2:: eine perspektivische Darstellung eines in Figur 1 dargestellten Im- plantatgrundkörpers zusammen mit einem ersten Befestigungsele- menthalteglied in der Einführ- oder Montagestellung;
- Figur 3:: eine perspektivische Darstellung des Implantatsystems aus Figur 1 mit einem zweiten Befestigungselementhalteglied in der Einführ- oder Montagestellung;
- Figur 4:: eine Rückansicht der in Figur 3 dargestellten Anordnung;
- Figur 5:: eine Seitenansicht eines Befestigungselements mit daran gela- gertem Befestigungselementhalteglied;
- Figur 6:: eine teilweise geschnittene Seitenansicht der Anordnung in Figur 5;
- Figur 7:: eine perspektivische Ansicht des Befestigungselementhalteglieds aus Figur 5 von oben;
- Figur 8:: eine perspektivische Ansicht des Befestigungselementhalteglieds aus Figur 7 von unten;
- Figur 9:: eine Seitenansicht eines weiteren Befestigungselementhalteglieds mit daran gelagertem Befestigungselement;
- Figur 10:: eine teilweise geschnittene Seitenansicht der Anordnung in Figur 9;
- Figur 11:: eine perspektivische Ansicht des Befestigungselementhalteglieds aus Figur 9 von oben;
- Figur 12:: eine perspektivische Ansicht des Befestigungselementhalteglieds aus Figur 11 von unten;
- Figur 13.: eine Draufsicht auf das in Figur 11 dargestellte Befestigungsele- menthalteglied; und
- Figur 14:: eine Seitenansicht des Befestigungselementhalteglieds aus Figur 13 in Richtung des Pfeils A.

Ein insgesamt mit dem Bezugszeichen 10 versehenes Implantatsystem zum Verbinden zweier Wirbelkörper, beispielsweise des 5. Lendenwirbels 12, der auch als "L5" bezeichnet wird, mit dem Kreuzbein 14 (Os sacrum), welches auch als "S1" bezeichnet wird, einer mit dem Bezugszeichen 16 versehenen menschlichen Wirbelsäule. Eine Fusion des 5. Lendenwirbels 12 und des Kreuzbeins 14 kommt insbesondere infolge einer degenerativen Erkrankung einer dazwischen liegenden Bandscheibe 18 in Betracht.

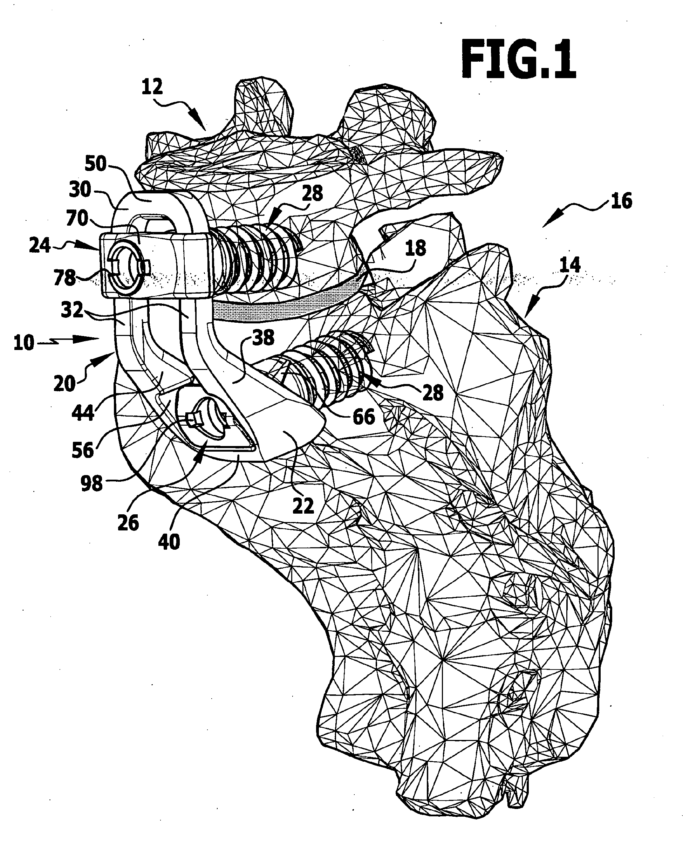

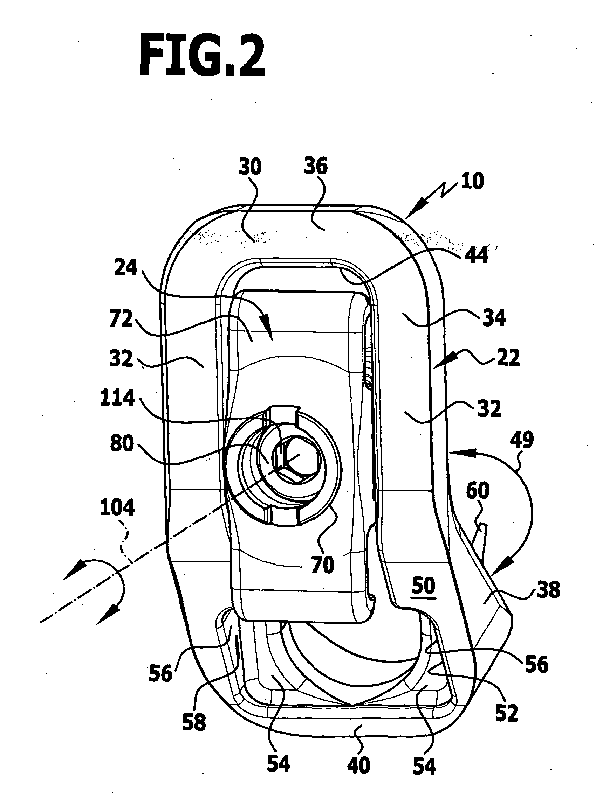

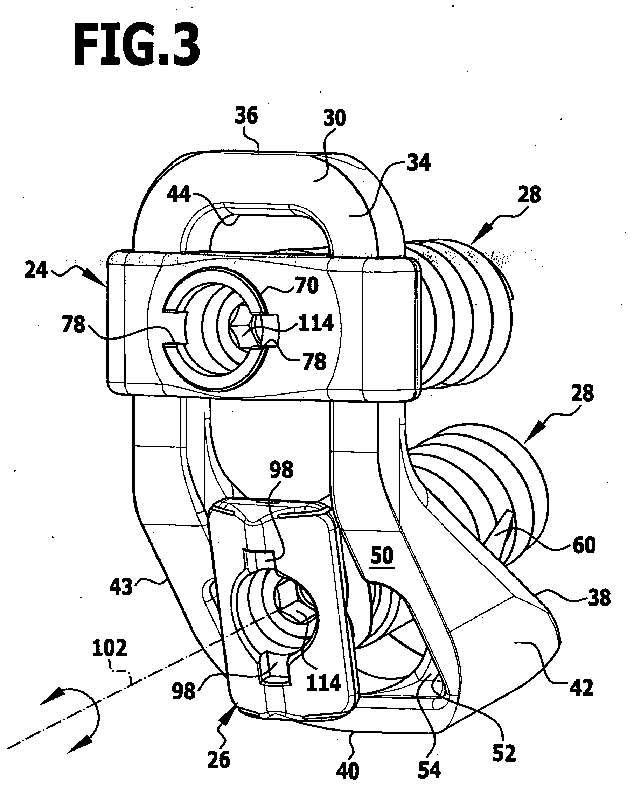

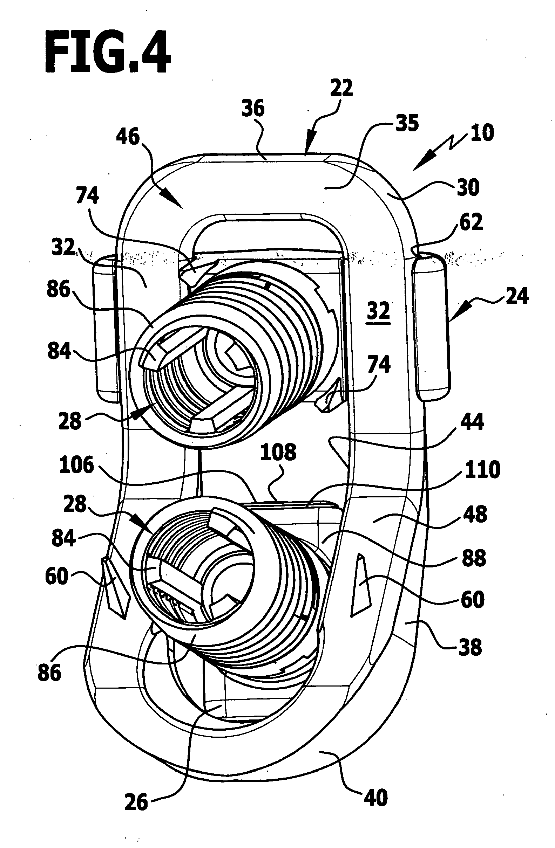

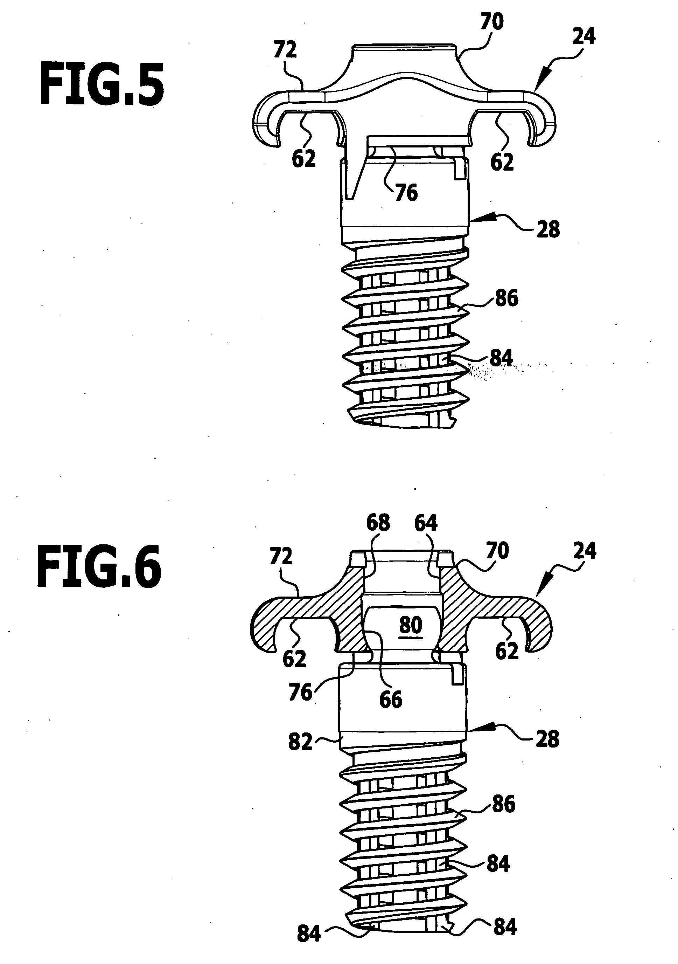

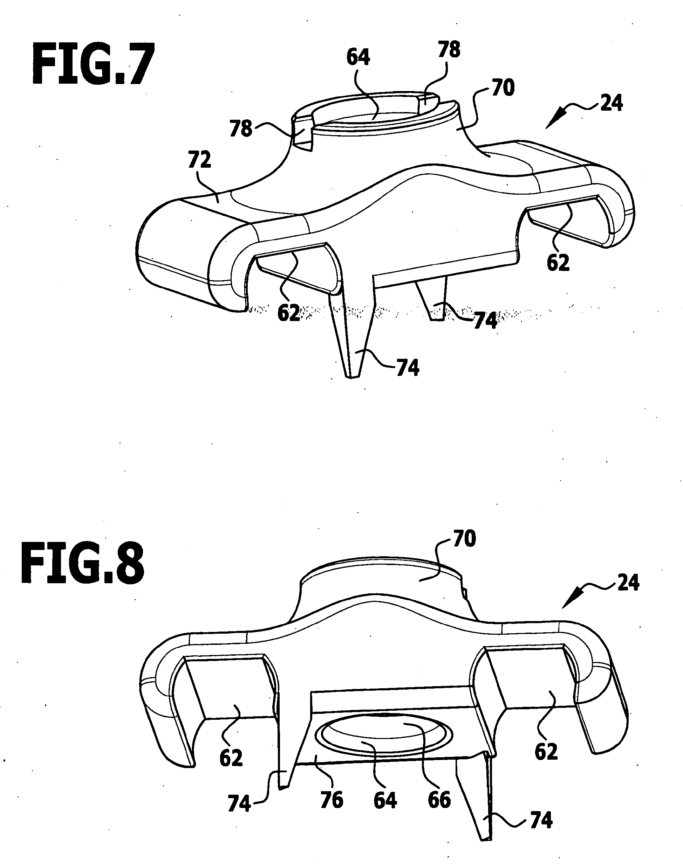

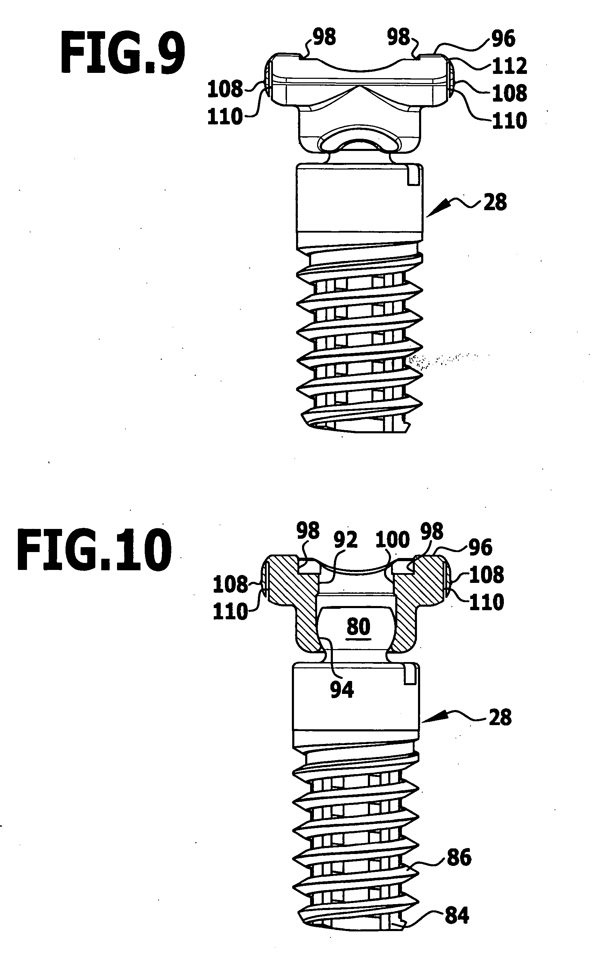

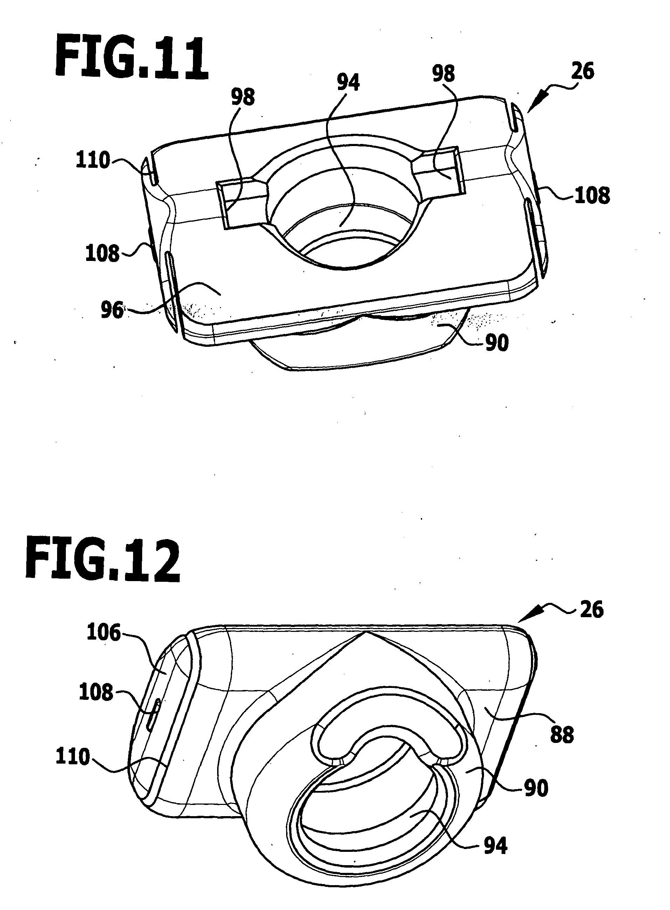

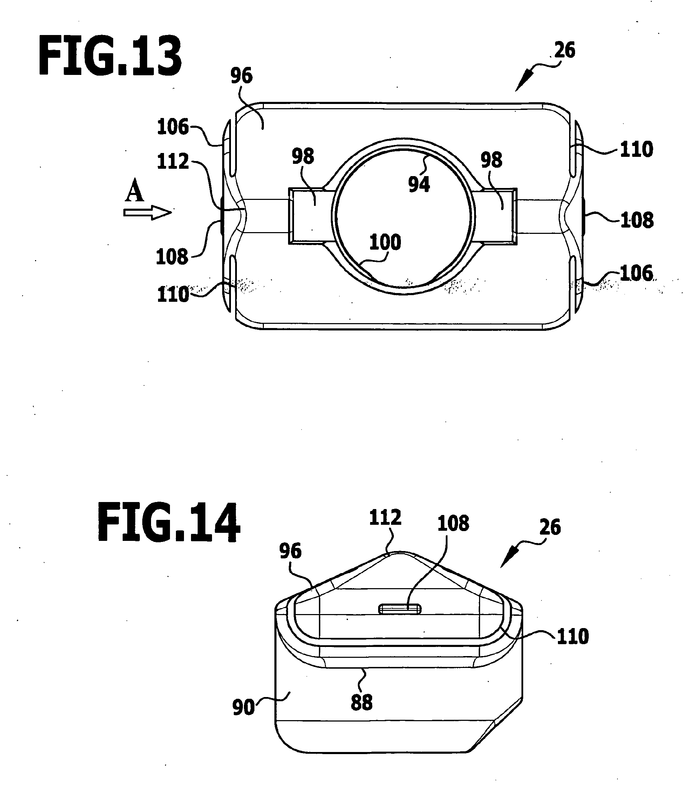

Das Implantatsystem 10 umfasst insbesondere mindestens ein Implantat 20 mit einem Implantatgrundkörper 22 und mit mindestens einem Befestigungselementhalteglied. Das in den Figuren 1 bis 14 dargestellte Implantatsystem 10 umfasst zwei Befestigungselementhalteglieder in Form eines oberen Schlittens 24 und eines unteren Schlittens 26. Des Weiteren umfasst das Implantatsystem 10 mindestens ein Befestigungselement, welches mindestens teilweise in einen Knochen oder Wirbelkörper eingebracht werden kann. Bei dem in den Figuren dargestellten Implantatsystem 10 sind beispielsweise zwei Befestigungselemente in Form von identisch ausgebildeten Knochenschrauben 28 vorgesehen.

Ferner kann das Implantatsystem 10 in den Figuren nicht dargestellte Halte-, Einsetz- und/oder Verbindungsinstrumente umfassen, um die beschriebenen Teile des Implantatsystems 10 in einen menschlichen oder tierischen Körper einzuführen und miteinander zu verbinden. In den Figuren auch nicht dargestellt sind Fixierelemente in Form von Sicherungsschrauben, mit denen die Schlitten 24 und 26 relativ zu den Knochenschrauben 28 optional festgelegt werden können.

Zur Stabilisierung einer Verbindung zwischen dem 5. Lendenwirbel 12 und dem Kreuzbein 14 dient der Implantatgrundkörper 22, der im Wesentlichen plattenförmig ausgebildet ist. Er umfasst einen oberen Teil 30, welcher zwei zueinander parallel verlaufende Verbindungsabschnitte 32 aufweist, die im Querschnitt rund, jedoch mit zwei Abflachungen 34 und 35 auf der Vorder- und Rückseite versehen sind, die jeweils in diametral entgegengesetzte Richtungen weisen. Die Verbindungsabschnitte sind über einen Querbügel 36 miteinander verbunden, der im Querschnitt entsprechend den Verbindungsabschnitten 32 geformt ist.

An den oberen Teil 30, der somit insgesamt im Wesentlichen U-förmig ausgebildet ist, schließt sich ein unterer Teil 38 des Implantatgrundkörpers 22 an, der in einer Draufsicht ebenfalls im Wesentlichen U-förmig ausgebildet ist. Der untere Teil weist symmetrisch zu einem Querverbinder 40 angeordnete Seitenelemente 42 und 43 auf, die einstückig mit den Verbindungsabschnitten 32 verbunden sind, so dass insgesamt eine langgestreckte, von den Verbindungsabschnitten 32, den Seitenelementen 42 und 43, dem Querbügel 36 und dem Querverbinder 40 begrenzte Durchbrechung 44 des Implantatgrundkörpers 22 ausgebildet wird. Die Seitenelemente 42 und 43 sind relativ zu den Verbindungsabschnitten 32 etwas abgewinkelt.

Die Abflachungen 35 bilden erste Flächenabschnitte einer insgesamt mit dem Bezugszeichen 46 versehenen Wirbelkörperanlagefläche. Rückwärtige Flächen des unteren Teils 38 bilden zweite Flächenabschnitte 48 der Wirbelkörperanlagefläche 46. Die zweiten Flächenabschnitte 48 gehen knickfrei in die Abfla chungen 35 über. Ferner schließen sie mit diesen einen Winkel von ungefähr 120° ein. Flächenabschnitte 50 auf einer Vorderseite des unteren Teils 38 gehen knickfrei in die Abflachungen 34 über. Sie sind zudem relativ zu den zweiten Flächenabschnitten 48 geneigt, und zwar in einem Winkel von etwa 20°. Eine Dicke des Implantatgrundkörpers 22 zwischen den zweiten Flächenabschnitten 48 und den Flächenabschnitten 50 nimmt ausgehend vom Übergangsbereich zwischen den zweiten Flächenabschnitten 48 und den Abflachungen 35 kontinuierlich zu, und zwar bis zum Querverbinder 40.

Im unteren Teil 38 ist auf der Vorderseite, die Flächenabschnitte 50 etwas verschmälernd, eine Ausnehmung 52 ausgebildet, welche im Wesentlichen quaderförmig ist. Verbliebenes Material des unteren Teils 38 bildet beidseits der Durchbrechung 44 Auflageflächen 54, die vorzugsweise parallel zu den Abflachungen 34 oder im Wesentlichen parallel zu diesen orientiert sind. Aufeinander zu weisende Seitenflächen 56 der Ausnehmung 52, die senkrecht zu einer von den Abflachungen 34 definierten Ebene orientiert sind, sind mit einer schlitzförmigen, parallel zu den Verbindungsabschnitten verlaufenden Vertiefung 58 versehen.

Ferner trägt der Implantatgrundkörper 22 zwei von den zweiten Flächenabschnitten 48 im Wesentlichen senkrecht abstehende, symmetrisch bezogen auf die Durchbrechung 44 angeordnete Dorne 60, die Knochen- oder Gewebeverankerungselemente bilden.

Der obere Schlitten 24 ist im Wesentlichen flach quaderförmig ausgebildet, mit nach außen weisenden abgerundeten Kanten. Auf einer Unterseite 76 sind symmetrisch zu einer Mittelebene zwei Nuten 62 ausgebildet, die im Wesentlichen korrespondierend zu den Verbindungsabschnitten 32 geformt sind. Offene Seiten der Nuten 62 sind etwas schmaler als eine maximale Breite der Verbindungsabschnitte 32.

Die Nuten 62 bilden zusammen mit den Verbindungsabschnitten 32 eine Verbindungsvorrichtung zum Verbinden des oberen Schlittens 24 mit dem Implantatgrundkörper 22. Die Verbindungsvorrichtung ist in Form einer Schnappverbindung ausgebildet, denn der obere Schlitten 24 kann insbesondere von oben auf den Implantatgrundkörper 22 aufgeclipst werden. Die Nuten 62 bilden zweite Verbindungsglieder in Form hinterschnittener Verbindungsgliedaufnahmen, die in einer Verbindungsstellung die Verbindungsabschnitte 32, die erste Verbindungsglieder bilden, in einem gesamten Winkelbereich von mehr als 180° umgreifen. Ferner ist es aufgrund der parallelen Anordnung der Verbindungsabschnitte 32 sowie der Nuten 62 möglich, den oberen Schlitten 24 in der Verbindungsstellung relativ zum Implantatgrundkörper 22 zu bewegen, nämlich zu verschieben, und zwar parallel zu einer von den Verbindungsabschnitten 32 definierten Längsrichtung.

Der obere Schlitten 24 ist ferner mit einer rotationssymmetrischen Durchbrechung 64 versehen, die in Richtung auf eine untere Seite des oberen Schlittens 24 in Form eines kalottenförmigen Sitzes 66 ausgebildet ist. An ihrem dem Sitz 66 gegenüberliegenden Ende ist die Durchbrechung mit einem Innengewinde 68 versehen, welches zu einem Außengewinde einer nicht dargestellten Klemmschraube ausgebildet ist. Das Innengewinde 68 ist im Bereich eines in etwa hülsenförmigen Führungsabschnitts 70 geformt, welcher von einer Oberseite 72 des oberen Schlittens 24 senkrecht absteht. Des Weiteren sind punktsymmetrisch zur Durchbrechung 64 und senkrecht von der Unterseite 76 des oberen Schlittens 24 weg weisend zwei abstehende Dorne 74 ausgebildet, die ebenfalls Knochen- oder Gewebeverankerungselemente bilden. Diametral gegenüberliegend sind am Führungsabschnitt 70 zwei nutförmige Aussparungen 78 vorgesehen, die parallel zu einer Längsrichtung des langgestreckten oberen Schlittens 24 orientiert sind. Sie bilden Werkzeugaufnahmen, um den oberen Schlitten 24 zu halten und/oder zu verdrehen.

Mit dem oberen Schlitten 24 kann die Knochenschraube 28 gelenkig verbunden werden. Letztere weist hierfür einen mindestens teilweise kalottenförmigen Kopf 80 auf, dessen Abmessungen zum Sitz 66 korrespondieren und mit diesem zusammen ein Kugelgelenk ausbilden. Der Kopf 80 ist vom Schraubenkörper 82 getrennt ausgebildet und mit diesem verschraubt. Zum losen Verbinden des oberen Schlittens 24 mit der Knochenschraube 28 wird zunächst der Kopf 80 mit einem sich daran anschließenden Gewindeabschnitt voran durch die Durchbrechung 64 vom Führungsabschnitt 70 her kommend hindurchgesteckt bis der Kopf 80 am Sitz anliegt. Anschließend wird der Schraubenkörper, welcher eine mit einem Innengewinde versehene Ausnehmung aufweist, mit dem Kopf 80 verschraubt. Der Schraubenkörper 82 ist nicht geschlossen und umfasst drei strebenartige, parallel zu einer Längsachse des Schraubenkörpers 82 ausgerichtete Streben 84, um die sich ein schraubenförmiges Gewinde 86 windet. Der Schraubenkörper 82 ist bestens dafür geeignet, in einen Knochen eingeschraubt zu werden. Ferner kann er im Wesentlichen vollständig von knöchernem Gewebe durchwachsen werden, was eine besonders gute und dauerhafte Verbindung mit einem Knochen, insbesondere einem Wirbelkörper, sicherstellt.

Der untere Schlitten 26 ist ebenfalls im Wesentlichen in Form einer quaderförmigen Platte ausgebildet, jedoch nur etwa zwei Drittel so lang, wie der obere Schlitten 24. Von einer Unterseite 88 des unteren Schlittens 26 erstreckt sich ein weiterer, im Wesentlichen hülsenförmig ausgebildeter Führungsabschnitt 90 senkrecht weg. Koaxial zum Führungsabschnitt 90 ist auch im unteren Schlitten 26 eine Durchbrechung 92 vorgesehen, die im Längsschnitt im Wesentlichen der Durchbrechung 64 entspricht. Sie ist derart geformt, dass im Bereich eines freien abstehenden Endes des Führungsabschnitts 90 im Innern ein Sitz 94 für den Kopf 80 einer weiteren Knochenschraube 28 ausgebildet ist.

Auf einer Oberseite 96 sind diametral gegenüberliegend beidseits der Durchbrechung 92 zwei quaderförmige Aussparungen 98 ausgebildet, die ebenfalls als Werkzeugaufnahmen zum Halten und/oder Verdrehen des unteren Schlittens 26 dienen. Die Durchbrechung 92 ist im Bereich der Aussparungen 98 mit einem Innengewinde 100 versehen, welches zu einem Außengewinde einer Klemmschraube korrespondiert, die in den Figuren nicht dargestellt ist. Sie dient dazu, eine Relativposition zwischen dem unteren Schlitten 26 und der Knochenschraube 28 durch Klemmung zu fixieren.

Der untere Schlitten 26 ist zudem derart ausgebildet, dass er in die Ausnehmung 52 im Wesentlichen formschlüssig eingesetzt werden kann. Die Auflageflächen 54 sind derart geformt, dass eine Längsachse 102 der Durchbrechung 92 im Wesentlichen parallel zu einer Längsachse 104 der Durchbrechung 64 ausgerichtet ist, wenn der obere Schlitten 24 auf die Verbindungsabschnitte 32 aufgeklipst ist und der untere Schlitten 26 in die Ausnehmung 52 eingesetzt ist.

Zum Verbinden des unteren Schlittens 26 mit dem Implantatgrundkörper 22 ist eine weitere Verbindungsvorrichtung in Form einer Rastverbindung vorgesehen. Erste Rastglieder bilden die Vertiefungen 58, die am Implantatgrundkörper 22 im Bereich der eine Verbindungselementhaltegliedaufnahmebildenden Ausnehmung 52 angeordnet sind. Voneinander in entgegengesetzter Richtung weg weisende Stirnflächen 106 des unteren Schlittens 26 tragen von ihnen abstehende Vorsprünge 108, die derart geformt sind, dass sie im Wesentlichen formschlüssig in die Vertiefungen 58 eintauchen können. Die Vorsprünge 108 bilden federnd gelagerte Rastvorsprünge, denn parallel zu den Stirnflächen 106 ist der untere Schlitten 26 von seiner Unterseite 88 her mit nutförmigen schmalen Schlitzen 110 versehen, so dass ein federartiges, den Vorsprung 108 tragendes Element ausgebildet wird, welches nur über einen schmalen Steg 112 mit der Oberseite 96 des unteren Schlittens 26 verbunden ist. Wird der untere Schlitten 26 mit dem Führungsabschnitt 90 voran quer zu den Verbindungsabschnitten 32 ausgerichtet und in die Ausnehmung 52 hinein bewegt, dann gleiten die Vorsprünge 108 an den Seitenflächen 56 auf, bis sie in die Vertiefungen 58 einfedern. Der untere Schlitten 26 ist dann mit dem Implantatgrundkörper 22 rastend verbunden.

Nachfolgend wird die Verwendung des Implantatsystems 10 näher erläutert in Verbindung mit einem Verfahren zum Verbinden zweier Wirbelkörper.

Falls die Knochenschrauben 28 noch nicht vormontiert mit den daran gelenkig gelagerten oberen und unteren Schlitten 24 und 26 geliefert werden, werden zunächst die Schlitten 24 und 26 mit jeweils einem Kopf 80 der Knochenschraube 28 verbunden. Jeder Kopf 80 weist jeweils eine Werkzeugaufnahme in Form eines koaxial zu seiner Symmetrieachse ausgerichteten Innensechskants 114 auf, so dass beispielsweise mit einem Sechskant-Schlüssel die Knochenschrauben 28 mit dem darin vormontierten Schlitten 24 beziehungsweise 26 in beispielsweise den 5. Lendenwirbel 12 oder das Kreuzbein 14 eingeschraubt werden können, und zwar im Wesentlichen parallel zueinander.

Als nächstes kann als optionaler Schritt eine Spreizvorrichtung an den Knochenschrauben 28 angebracht werden, um den Lendenwirbel 12 und das Kreuzbein 14 zu distrahieren und die geschädigte Bandscheibe 18 zu bearbeiten.

Ist die Bandscheibe 18 behandelt, beispielsweise ausgeräumt oder teilreseziert, und wurden der Lendenwirbel 12 und das Kreuzbein 14 in eine gewünschte Relativposition zueinander gebracht, kann in einem nächsten Schritt der Implantatkörper 22 mit den Knochenschrauben 28 verbunden werden. Zu diesem Zweck werden die Schlitten 24 und 26 parallel zu ihren Längsrichtungen ausgerichtet, das heißt die Nuten 62 und die Schlitze 110 verlaufen dann im Wesentlichen parallel zueinander. In dieser Einführ- oder Montagestellung kann der Implantatgrundkörper 22 über die Schlitten 24 und 26 geschoben werden, und zwar dadurch, dass diese durch die dafür ausreichend groß vorgesehene Durchbrechung 64 durchgeschoben werden. Alternativ kann man auch davon reden, dass der Implantatgrundkörper 22 über die an den Knochenschrauben 28 gelagerten und ausgerichteten Schlitten 24 und 26 geschoben wird.

Um die Schlitten 24 und 26 mit dem Implantatgrundkörper 22 verbinden zu können, müssen diese jeweils um die Längsachsen 102 und 104 um 90° gedreht werden. Wird der Implantatgrundkörper 22 dann in von den Schraubenkörpern 82 weg weisender Richtung bewegt, können die Verbindungsabschnitte 32 in die Nuten 62 einrasten und der untere Schlitten 26 in die Ausnehmung 52 eingeführt werden, bis die Vorsprünge 108 in die Vertiefungen 58 einrasten. Eine relative Justierung ist zwischen dem oberen Schlitten 24 und dem Implantatgrundkörper 22 möglich, denn der obere Schlitten 24 ist auf den Verbindungsabschnitten 32 in dieser Verbindungsstellung verschiebbar gelagert.

Anschließend können die Knochenschrauben weiter bis in ihre endgültige Position in den Lendenwirbel 12 und in das Kreuzbein 14 eingeschraubt werden. Die Köpfe 80 sind zum weiteren Einschrauben der Knochenschrauben 28 weiterhin durch die Durchbrechungen 64 und 92 zugänglich. Die Knochenschrauben 28 werden so weit in den Lendenwirbel 12 und in das Kreuzbein 14 eingetrieben, bis die Wirbelkörperanlagefläche 46 im Wesentlichen vollständig am Lendenwirbel 12 und am Kreuzbein 14 anliegt, das heißt die Abflachungen 35 am Lendenwirbel 12 und die zweiten Flächenabschnitte 48 am Kreuzbein 14. Zur endgültigen Fixierung der Schlitten 24 und 26 an den Knochenschrauben 28 werden Klemmschrauben in die Schlitten 24 und 26 eingeschraubt, und zwar in die Innengewinde 68 und 100, bis diese den Kopf 80 im jeweiligen Sitz 66 beziehungsweise 94 klemmend halten.

Bei einer eventuell erforderlichen Revision oder Entnahme des Implantatsystems 10 wird in umgekehrter Reihenfolge vorgegangen und das Implantatsystem explantiert.

Besonders vorteilhaft ist es, dass das Implantatsystem 10 durch einen einzigen ventralen Zugang eingesetzt werden kann. Ein dorsaler Zugang ist nicht erforderlich.

Der Implantatgrundkörper 22 und die Schlitten 24 und 26 sind vorzugsweise einstückig und aus einem körperverträglichen Material, beispielsweise Titan oder einem körperverträglichen Kunststoff ausgebildet. Anstelle der oben beschriebenen Knochenschrauben 28 können auch andere, üblicherweise verwendete Knochenschrauben zum Einsatz kommen. Diese sollten jedoch vorzugsweise einen zumindest teilweise kalottenförmigen Kopf aufweisen.

Alternativ wäre es denkbar, den oberen Teil 30 und den unteren Teil 38 des Implantatgrundkörpers 22 gelenkig zu verbinden.

## Patentansprüche

1. Implantat (20) zum Verbinden zweier Wirbelkörper (12, 14) einer menschlichen oder tierischen Wirbelsäule (16) umfassend einen Implantatgrundkörper (22) und mindestens ein Befestigungselementhalteglied (24, 26), an welchem mindestens ein Teil (80) eines mindestens teilweise in einen Wirbelkörper (12, 14) einbringbaren Befestigungselements (28) anorden-, lager- und/oder festlegbar ist, wobei das mindestens eine Befestigungselementhalteglied (24, 26) und der Implantatgrundkörper (22) miteinander lösbar verbindbar sind, wobei in einer Montagestellung das mindestens eine Befestigungselementhalteglied (24, 26) und der Implantatgrundkörper (22) voneinander getrennt und in einer Verbindungsstellung miteinander verbunden sind, wobei eine Verbindungsvorrichtung vorgesehen ist zum Verbinden des Implantatgrundkörpers (22) und des mindestens einen Befestigungselementhalteglieds (24, 26) in der Verbindungsstellung, wobei die Verbindungsvorrichtung mindestens ein erstes und mindestens ein zweites, mit dem mindestens einen ersten Verbindungsglied (32; 58) in Eingriff bringbares Verbindungsglied (62; 108) umfasst, wobei das mindestens eine erste und das min- H destens eine zweite Verbindungsglied (32, 62; 58, 108) in der Verbindungsstellung in Eingriff stehen, wobei das mindestens eine erste Verbindungsglied (32; 58) am Implantatgrundkörper und das mindestens eine zweite Verbindungsglied (62; 108) am mindestens einen Befestigungselementhalteglied (24, 26) angeordnet ist, **dadurch gekennzeichnet, dass** die Verbindungsvorrichtung in Form einer Rast- oder Schnappverbindung mit mindestens einem ersten Rastglied (58) und mindestens einem zweiten Rastglied (108) ausgebildet ist und dass das mindestens eine Befestigungselementhalteglied (24) in der Verbindungsstellung relativ zum Implantatgrundkörper (22) bewegbar ist.

2. Implantat nach Anspruch 1, **dadurch gekennzeichnet, dass** zwei mit dem Implantatgrundkörper (22) lösbar verbindbare Befestigungselementhalteglieder (24, 26) vorgesehen sind.

3. Implantat nach Anspruch 1 oder 2, **dadurch gekennzeichnet, dass** das mindestens eine Befestigungselementhalteglied (24) in der Verbindungsstellung relativ zum Implantatgrundkörper (22) verschiebbar ist.

4. Implantat nach einem der voranstehenden Ansprüche, **dadurch gekennzeichnet, dass** das mindestens eine Befestigungselementhalteglied (26) in der Verbindungsstellung am Implantatgrundkörper (22) unbeweglich gehalten ist.

5. Implantat nach einem der voranstehenden Ansprüche, **dadurch gekennzeichnet, dass** das mindestens eine erste Rastglied (58) in Form einer Rastausnehmung (58) ausgebildet ist und dass das mindestens eine zweite Rastglied (108) in Form eines federnd gelagerten Rastvorsprungs (108) ausgebildet ist.

6. Implantat nach einem der voranstehenden Ansprüche, **dadurch gekennzeichnet, dass** am Implantatgrundkörper eine Befestigungselementhaltegliedaufnahme (52) ausgebildet ist, in welche das mindestens eine Befestigungselementhalteglied (26) in der Verbindungsstellung mindestens teilweise eintaucht und/oder gelagert ist.

7. Implantat nach Anspruch 6, **dadurch gekennzeichnet, dass** das mindestens eine erste oder das mindestens eine zweite Verbindungsglied (58) der Verbindungsvorrichtung an der Befestigungselementhaltegliedaufnahme (52) angeordnet ist.

8. Implantat nach einem der voranstehenden Ansprüche, **dadurch gekennzeichnet, dass** das mindestens eine zweite Verbindungsglied (62) in Form einer hinterschnittenen Verbindungsgliedaufnahme (62) und dass das mindestens eine erste Verbindungsglied (32) in Form eines zur Verbindungsgliedaufnahme (62) im Querschnitt korrespondierenden Verbindungsabschnitts (32) ausgebildet ist, welcher in der Verbindungsstellung von der Verbindungsgliedaufnahme (62) in einem gesamten Winkelbereich von mehr als 180° umgriffen wird.

9. Implantat nach Anspruch 8, **dadurch gekennzeichnet, dass** der Verbindungsabschnitt (32) stabförmig und im Querschnitt rund, oval, rechteckig, unrund oder im Wesentlichen rund, oval, rechteckig oder unrund geformt ist.

10. Implantat nach einem der voranstehenden Ansprüche, **dadurch gekennzeichnet, dass** zwei erste und zwei zweite Verbindungsglieder (32, 62; 58, 108) vorgesehen sind und dass die zwei ersten und/oder die zwei zweiten Verbindungsglieder (32, 62; 58, 108) parallel oder im Wesentlichen parallel zueinander ausgerichtet sind.

11. Implantat nach einem der voranstehenden Ansprüche, **dadurch gekennzeichnet, dass** der Implantatgrundkörper (22) mindestens eine Einführdurchbrechung (44) aufweist, die derart bemessen ist, dass das mindestens eine Befestigungselementhalteglied (24, 26) nur in einer Einführstellung oder in der Montagestellung durch die mindestens eine Einführdurchbrechung (44) einführbar ist, in welcher Längsachsen des Implantatgrundkörpers (22) und des mindestens einen Befestigungselementhalteglieds (24, 26) relativ zueinander in einem vorgegebenen Winkelbereich ausgerichtet sind.

12. Implantat nach Anspruch 11, **dadurch gekennzeichnet, dass** die Einführdurchbrechung (44) langgestreckt oder in Form eines Langlochs (44) ausgebildet ist.

13. Implantat nach einem der voranstehenden Ansprüche, **dadurch gekennzeichnet, dass** das mindestens eine Befestigungselementhalteglied (24, 26) eine Befestigungselementaufnahme (64, 92) für ein Befestigungselement (28) aufweist.

14. Implantat nach Anspruch 13, **dadurch gekennzeichnet, dass** mindestens zwei Befestigungselementhalteglieder (24, 26) vorgesehen sind und dass mindestens zwei der mindestens zwei Befestigungselementhalteglieder (24, 26) Befestigungselementaufnahmen (64, 92) aufweisen, deren Längsachsen (102, 104) in der Verbindungsstellung parallel oder im Wesentlichen parallel zueinander ausgerichtet sind.

15. Implantat nach einem der Ansprüche 13 oder 14, **dadurch gekennzeichnet, dass** die Befestigungselementaufnahme (64, 92) in Form einer Durchbrechung (64, 92) ausgebildet ist.

16. Implantat nach einem der Ansprüche 13 bis 15, **dadurch gekennzeichnet, dass** die Befestigungselementaufnahme (64, 92) einen Lagersitz (66, 94) für einen Gelenkkopf (80) eines Befestigungselements (28) umfasst.

17. Implantat nach einem der Ansprüche 13 bis 16 **dadurch gekennzeichnet, dass** sich an die Befestigungselementaufnahme (64, 92) mindestens ein Führungsabschnitt (70, 90) anschließt.

18. Implantat nach Anspruch 17, **dadurch gekennzeichnet, dass** der mindestens eine Führungsabschnitt (70, 90) hülsenförmig oder im Wesentlichen hülsenförmig ausgebildet ist.

19. Implantat nach einem der voranstehenden Ansprüche, **dadurch gekennzeichnet, dass** das mindestens eine Befestigungselementhalteglied (24) und/oder der Implantatgrundkörper (22) mindestens ein Knochen- oder Gewebeverankerungselement (60, 74) aufweisen.

20. Implantat nach Anspruch 19, **dadurch gekennzeichnet, dass** das mindestens eine Knochen- oder Gewebeverankerungselement (60, 74) in Form eines abstehenden Dorns (60, 74) ausgebildet ist.

21. Implantat nach einem der voranstehenden Ansprüche, **dadurch gekennzeichnet, dass** der Implantatgrundkörper (22) eine äußere Wirbelkörperanlagefläche (46) aufweist, dass die Wirbelkörperanlagefläche (46) mindestens zwei Flächenabschnitte (35, 48) aufweist und dass die Flächenabschnitte (35, 48) relativ zueinander um einen Anlagewinkel (49) geneigt sind.

22. Implantat nach Anspruch 21, **dadurch gekennzeichnet, dass** der Anlagewinkel (49) einen Wert in einem Bereich von 20° bis 80° aufweist, vorzugsweise einen Wert von 40° bis 60°.

23. Implantat nach einem der Ansprüche 21 oder 22, **dadurch gekennzeichnet, dass** der Implantatgrundkörper (22) einen ersten und einen mit dem ersten gelenkig verbundenen zweiten Teil (30, 38) umfasst und dass der erste und der zweite Teil (30, 38) jeweils einen Flächenabschnitt (35, 48) definieren.

24. Implantat nach einem der Ansprüche 21 bis 23, **dadurch gekennzeichnet, dass** die Wirbelkörperanlagefläche (46) die mindestens eine Einführdurchbrechung (44) mindestens teilweise begrenzt.

25. Implantat nach einem der Ansprüche 21 bis 24, **dadurch gekennzeichnet, dass** die Wirbelkörperanlagefläche (46) von der mindestens einen Befestigungselementhaltegliedaufnahme (52) in entgegengesetzter Richtung weg weist.

26. Implantat nach einem der voranstehenden Ansprüche, **dadurch gekennzeichnet, dass** das mindestens eine Befestigungselementhalteglied (24, 26) mindestens eine Werkzeugaufnahme (78, 98) für ein Halte- und/oder Einsetzinstrument aufweist.

27. Implantat nach einem der voranstehenden Ansprüche, **gekennzeichnet durch** mindestens zwei mindestens teilweise in einen Wirbelkörper (12, 14) einbringbaren Befestigungselemente (28).

28. Implantat nach Anspruch 27, **dadurch gekennzeichnet, dass** mindestens eines der Befestigungselementhalteglieder (24, 26) in einer Einsetzstellung an einem der Befestigungselemente (28) beweglich gelagert ist und dass in einer Verbindungsstellung das mindestens eine Befestigungselementhalteglied (24, 26) unbeweglich an dem einen der Befestigungselemente (28) festgelegt ist.

29. Implantat nach Anspruch 27 oder 28, **dadurch gekennzeichnet, dass** mindestens eines der Befestigungselemente (28) in Form einer Knochenschraube (28) ausgebildet ist.

30. Implantat nach Anspruch 29, **dadurch gekennzeichnet, dass** die Knochenschraube (28) in Form einer Polyaxialschraube (28) ausgebildet ist, die einen Gelenkkopf (80) umfasst und dass mindestens eines der Befestigungselementhalteglieder (24, 26) eine zum Gelenkkopf (80) korrespondierende Gelenkkopfaufnahme (66, 94) für den Gelenkkopf (80) aufweist.

31. Implantat nach einem der Ansprüche 27 bis 30, **dadurch gekennzeichnet, dass** mindestens eines der Befestigungselemente (28) mit einem Fixierelement oder Sicherungsglied an dem mindestens einen Befestigungselementhalteglied (24, 26) festlegbar ist.

32. Implantat nach einem der Ansprüche 27 bis 31, **dadurch gekennzeichnet, dass** das Implantatsystem (10) ein Instrumentarium zum Einsetzen der Befestigungselemente (28) in einen Wirbelkörper (12, 14) und/oder zum Festlegen mindestens eines Befestigungselements (28) an mindestens einem Befestigungselementhalteglied (24, 26) umfasst.

## Claims

1. Implant (20) for joining two vertebral bodies (12, 14) in the vertebral column (16) of a human being or an animal, comprising a main implant body (22) and at least one fastener element holding member (24, 26) on which at least one part (80) of a fastener element (28) which is insertable at least partially into a vertebral body (12, 14) can be arranged, mounted and/or secured, the at least one fastener element holding member (24, 26) and the main implant body (22) being releasably connectable to each other, the at least one fastener element holding member (24, 26) and the main implant body (22) being separate from each other in an assembly position and being connected to each other in a connected position, a connecting device being provided for connecting the main implant body (22) and the at least one fastener element holding member (24, 26) in the connected position, the connecting device comprising at least one first and at least one second connecting member (62; 108) which can engage with the at least one first connecting member (32; 58), the at least one first and the at least one second connecting members (32, 62; 58, 108) being in engagement in the connected position, the at least one first connecting member (32; 58) being arranged on the main implant body, and the at least one second connecting member (62; 108) on the at least one fastener element holding member (24, 26), **characterized in that** the connecting device is configured as a locking or snap-in connection with at least one first locking member (58) and at least one second locking member (108), and **in that** the at least one fastener element holding member (24) is movable relative to the main implant body (22) in the connected position.

2. Implant in accordance with claim 1, **characterized in that** two fastener element holding members (24, 26) which are releasably connectable to the main implant body (22) are provided.

3. Implant in accordance with claim 1 or 2, **characterized in that** the at least one fastener element holding member (24) is displaceable relative to the main implant body (22) in the connected position.

4. Implant in accordance with any one of the preceding claims, **characterized in that** the at least one fastener element holding member (26) is held immovably on the main implant body (22) in the connected position.

5. Implant in accordance with any one of the preceding claims, **characterized in that** the at least one first locking member (58) is in the form of a locking recess (58), and **in that** the at least one second locking member (108) is in the form of a resiliently mounted locking projection (108).

6. Implant in accordance with any one of the preceding claims, **characterized in that** there is formed on the main implant body a fastener element holding member receptacle (52), into which the at least one fastener element holding member (26) enters at least partially and/or in which the at least one fastener element holding member (26) is mounted in the connected position.

7. Implant in accordance with claim 6, **characterized in that** the at least one first or the at least one second connecting member (58) of the connecting device is arranged on the fastener element holding member receptacle (52).

8. Implant in accordance with any one of the preceding claims, **characterized in that** the at least one second connecting member (62) is in the form of an undercut connecting member receptacle (62), and **in that** the at least one first connecting member (32) is in the form of a connecting section (32) which corresponds in cross section to the connecting member receptacle (62) and in the connected position is embraced by the connecting member receptacle (62) over a total angular range of more than 180°.

9. Implant in accordance with claim 8, **characterized in that** the connecting section (32) is rod-shaped and is of round, oval, rectangular, non-circular or substantially round, oval, rectangular or non-circular cross-sectional shape.

10. Implant in accordance with any one of the preceding claims, **characterized in that** two first and two second connecting members (32, 62; 58, 108) are provided, and **in that** the two first and/or the two second connecting members (32, 62; 58, 108) are aligned parallel or substantially parallel to each other.

11. Implant in accordance with any one of the preceding claims, **characterized in that** the main implant body (22) comprises an insertion through-opening (44) which is of such dimensions that the at least one fastener element holding member (24, 26) is only insertable through the at least one insertion through-opening (44) in an insertion position or in the assembly position, in which longitudinal axes of the main implant body (22) and of the at least one fastener element holding member (24, 26) are aligned in a prescribed angular range relative to each other.

12. Implant in accordance with claim 11, **characterized in that** the insertion through-opening (44) is of elongated configuration or is in the form of an oblong hole (44).

13. Implant in accordance with any one of the preceding claims, **characterized in that** the at least one fastener element holding member (24, 26) comprises a fastener element receptacle (64, 92) for a fastener element (28).

14. Implant in accordance with claim 13, **characterized in that** at least two fastener element holding members (24, 26) are provided, and **in that** at least two of the at least two fastener element holding members (24, 26) comprise fastener element receptacles (64, 92) whose longitudinal axes (102, 104) are aligned parallel or substantially parallel to each other in the connected position.

15. Implant in accordance with claim 13 or 14, **characterized in that** the fastener element receptacle (64, 92) is in the form of a through-opening (64, 92).

16. Implant in accordance with any one of claims 13 to 15, **characterized in that** the fastener element receptacle (64, 92) comprises a bearing seat (66, 94) for a joint head (80) of a fastener element (28).

17. Implant in accordance with any one of claims 13 to 16, **characterized in that** at least one guide section (70, 90) adjoins the fastener element receptacle (64, 92).

18. Implant in accordance with claim 17, **characterized in that** the at least one guide section (70, 90) is of sleeve-shaped or substantially sleeve-shaped configuration.

19. Implant in accordance with any one of the preceding claims, **characterized in that** the at least one fastener element holding member (24) and/or the main implant body (22) comprise at least one bone or tissue anchoring element (60, 74).

20. Implant in accordance with claim 19, **characterized in that** the at least one bone or tissue anchoring element (60, 74) is in the form of a protruding spike (60, 74).

21. Implant in accordance with any one of the preceding claims, **characterized in that** the main implant body (22) comprises an outer vertebral body engaging surface (46), **in that** the vertebral body engaging surface (46) comprises at least two surface sections (35, 48), and **in that** the surface sections (35, 48) are inclined at an engagement angle (49) relative to each other.

22. Implant in accordance with claim 21, **characterized in that** the engagement angle (49) has a value ranging from 20° to 80°, preferably a value of 40° to 60°.

23. Implant in accordance with claim 21 or 22, **characterized in that** the main implant body (22) comprises a first part (30) and a second part (38) articulatedly connected to the first part, and **in that** the first and second parts (30, 38) each define a surface section (35, 48).

24. Implant in accordance with any one of claims 21 to 23, **characterized in that** the vertebral body engaging surface (46) delimits at least partially the at least one insertion through-opening (44).

25. Implant in accordance with any one of claims 21 to 24, **characterized in that** the vertebral body engaging surface (46) faces away from the at least one fastener element holding member receptacle (52) in the opposite direction.

26. Implant in accordance with any one of the preceding claims, **characterized in that** the at least one fastener element holding member (24, 26) comprises at least one tool receptacle (78, 98) for a holding and/or insertion instrument.

27. Implant in accordance with any one of the preceding claims, **characterized by** at least two fastener elements (28) which are at least partially insertable into a vertebral body (12, 14).

28. Implant in accordance with claim 27, **characterized in that** at least one of the fastener element holding members (24, 26) is movably mounted on one of the fastener elements (28) in an insertion position, and **in that** the at least one fastener element holding member (24, 26) is immovably secured to the one of the fastener elements (28) in a connected position.

29. Implant in accordance with claim 27 or 28, **characterized in that** at least one of the fastener elements (28) is in the form of a bone screw (28).

30. Implant in accordance with claim 29, **characterized in that** the bone screw (28) is in the form of a polyaxial screw (28) having a joint head (80), and **in that** at least one of the fastener element holding members (24, 26) comprises a joint head receptacle (66, 94) for the joint head (80), which corresponds to the joint head (80).

31. Implant in accordance with any one of claims 27 to 30, **characterized in that** at least one of the fastener elements (28) is securable with a fixing element or securing member to the at least one fastener element holding member (24, 26).

32. Implant in accordance with any one of claims 27 to 31, **characterized in that** the implant system (10) comprises instruments for inserting the fastener elements (28) into a vertebral body (12, 14) and/or for securing at least one fastener element (28) to at least one fastener element holding member (24, 26).

## Revendications

1. Implant (20) destiné à relier deux corps de vertèbre (12, 14) d'une colonne vertébrale (16) humaine ou animale, comprenant un corps de base d'implant (22) et au moins un organe de maintien d'élément de fixation (24, 26) sur lequel peut être agencée, montée et/ou fixée au moins une partie (80) d'un élément de fixation (28) pouvant être introduit au moins partiellement dans un corps de vertèbre (12, 14), implant
dans lequel ledit au moins un organe de maintien d'élément de fixation (24, 26) et le corps de base d'implant (22) peuvent être reliés mutuellement de manière amovible,
dans lequel, dans une position de montage, ledit au moins un organe de maintien d'élément de fixation (24, 26) et le corps de base d'implant (22) sont séparés l'un de l'autre et, dans une position de liaison, sont reliés l'un à l'autre,
dans lequel est prévu un dispositif de liaison pour relier le corps de base d'implant (22) et ledit au moins un organe de maintien d'élément de fixation (24, 26) dans la position de liaison,
dans lequel le dispositif de liaison comprend au moins un premier et au moins un deuxième organe de liaison (62 ; 108), le deuxième pouvant être amené en prise avec ledit au moins un premier organe de liaison (32 ; 58), dans lequel ledit au moins un premier et ledit au moins un deuxième organe de liaison (32, 62 ; 58, 108) sont en prise réciproque dans la position de liaison, et
dans lequel ledit au moins un premier organe de liaison (32 ; 58) est agencé sur le corps de base d'implant, et ledit au moins un deuxième organe de liaison (62 ; 108) est agencé sur ledit au moins un organe de maintien d'élément de fixation (24, 26),
**caractérisé en ce que** le dispositif de liaison est réalisé sous la forme d'une liaison par encliquetage ou à déclic avec au moins un premier organe d'encliquetage (58) et au moins un deuxième organe d'encliquetage (108), et **en ce que** ledit au moins un organe de maintien d'élément de fixation (24) est, dans la position de liaison, mobile par rapport au corps de base d'implant (22) .

2. Implant selon la revendication 1, **caractérisé en ce que** sont prévus deux organes de maintien d'élément de fixation (24, 26) pouvant être reliés de manière amovible au corps de base d'implant (22).

3. Implant selon la revendication 1 ou la revendication 2, **caractérisé en ce que** ledit au moins un organe de maintien d'élément de fixation (24) peut, dans la position de liaison, coulisser par rapport au corps de base d' implant (22).

4. Implant selon l'une des revendications précédentes, **caractérisé en ce que** ledit au moins un organe de maintien d'élément de fixation (26) est, dans la position de liaison, maintenu non mobile sur le corps de base d' implant (22).

5. Implant selon l'une des revendications précédentes, **caractérisé en ce que** ledit au moins un premier organe d'encliquetage (58) est réalisé sous la forme d'un évidement d'encliquetage (58), et **en ce que** ledit au moins un deuxième organe d'encliquetage (108) est réalisé sous la forme d'une protubérance d'encliquetage (108) à montage élastique.

6. Implant selon 1"une des revendications précédentes, **caractérisé en ce que** sur le corps de base d'implant est formé un logement d'organe de maintien d'élément de fixation (52), dans lequel s'engage au moins partiellement et/ou est monté ledit au moins un organe de maintien d'élément de fixation (26), dans la position de liaison.

7. Implant selon la revendication 6, **caractérisé en ce que** ledit au moins un premier ou ledit au moins un deuxième organe de liaison (58) du dispositif de liaison est agencé au niveau du logement d'organe de maintien d'élément de fixation (52).

8. Implant selon l'une des revendications précédentes, **caractérisé en ce que** ledit au moins un deuxième organe de liaison (62) est réalisé sous la forme d'un logement d'organe de liaison (62) en contre-dépouille, et **en ce que** ledit au moins un premier organe de liaison (32) est réalisé sous la forme d'un tronçon de liaison (32) qui, en section transversale, correspond au logement d'organe de liaison (62), et qui, dans la position de liaison, est entouré par le logement d'organe de liaison (62) sur une plage angulaire totale de plus de 180°.

9. Implant selon la revendication 8, **caractérisé en ce que** le tronçon de liaison (32) présente une forme de barre et est, en section transversale, de forme ronde, ovale, rectangulaire, non circulaire, ou bien de forme sensiblement ronde, ovale, rectangulaire ou non circulaire.

10. Implant selon l'une des revendications précédentes, **caractérisé en ce que** sont prévus deux premiers et deux deuxièmes organes de liaison (32, 62 ; 58, 108), et **en ce que** les deux premiers et/ou les deux deuxièmes organes de liaison (32, 62 ; 58, 108) sont orientés parallèlement ou sensiblement parallèlement l'un à l'autre.

11. Implant selon l'une des revendications précédentes, **caractérisé en ce que** le corps de base d'implant (22) présente au moins un passage ajouré d'introduction (44) qui est dimensionné de façon telle, que ledit au moins un organe de maintien d'élément de fixation (24, 26) ne puisse être introduit à travers le passage ajouré d'introduction (44) que dans une position d'introduction ou dans la position de montage, dans laquelle des axes longitudinaux du corps de base d'implant (22) et dudit au moins un organe de maintien d'élément de fixation (24, 26) sont orientés l'un par rapport à l'autre selon un angle situé dans une plage angulaire prédéterminée.

12. Implant selon la revendication 11, **caractérisé en ce que** le passage ajouré d'introduction (44) est d'une configuration allongée ou présente une forme de trou oblong (44).

13. Implant selon l'une des revendications précédentes, **caractérisé en ce que** ledit au moins un organe de maintien d'élément de fixation (24, 26) présente un logement d'élément de fixation (64, 92) pour un élément de fixation (28).

14. Implant selon la revendication 13, **caractérisé en ce que** sont prévus au moins deux organes de maintien d'élément de fixation (24, 26), et **en ce qu'**au moins deux des dits au moins deux organes de maintien d'élément de fixation (24, 26) présentent des logements d'élément de fixation (64, 92) dont les axes longitudinaux (102, 104) sont, dans la position de liaison, orientés de manière parallèle ou sensiblement parallèle l'un à l'autre.

15. Implant selon l'une des revendications 13 ou 14, **caractérisé en ce que** le logement d'élément de fixation (64, 92) est réalisé sous la forme d'un passage ajouré (64, 92).

16. Implant selon l'une des revendications 13 à 15, **caractérisé en ce que** le logement d'élément de fixation (64, 92) englobe une portée de montage (66, 94) pour une tête d'articulation (80) d'un élément de fixation (28).

17. Implant selon l'une des revendications 13 à 16, **caractérisé en ce qu'**au logement d'élément de fixation (64, 92) se raccorde au moins un tronçon de guidage (70, 90) .

18. Implant selon la revendication 17, **caractérisé en ce que** ledit au moins un tronçon de guidage (70, 90) est d'une configuration en forme de douille ou sensiblement en forme de douille.

19. Implant selon l'une des revendications précédentes, **caractérisé en ce que** ledit au moins un organe de maintien d'élément de fixation (24) et/ou le corps de base d'implant (22) présentent au moins un élément d'ancrage dans l'os ou les tissus (60, 74).

20. Implant selon la revendication 19, **caractérisé en ce que** ledit au moins un élément d'ancrage dans l'os ou les tissus (60, 74) est réalisé sous la forme d'une pointe (60, 74) en saillie.

21. Implant selon l'une des revendications précédentes, **caractérisé en ce que** le corps de base d'implant (22) présente une surface d'appui extérieure de corps de vertèbre (46), **en ce que** la surface d'appui de corps de vertèbre (46) présente au moins deux tronçons de surface (35, 48), et **en ce que** les tronçons de surface (35, 48) sont inclinés mutuellement l'un par rapport à l'autre d'un angle d'appui (49).

22. Implant selon la revendication 21, **caractérisé en ce que** l'angle d'appui (49) présente une valeur située dans une plage de 20° à 80°, de préférence une valeur de 40° à 60°.

23. Implant selon l'une des revendications 21 ou 22, **caractérisé en ce que** le corps de base d'implant (22) comprend une première et une deuxième partie (30, 38), la deuxième partie étant reliée de manière articulée à la première, et **en ce que** la première et la deuxième partie (30, 38) définissent chacune respectivement un tronçon de surface (35, 48).

24. Implant selon l'une des revendications 21 à 23, **caractérisé en ce que** la surface d'appui de corps de vertèbre (46) délimite au moins partiellement ledit au moins un passage ajouré d'introduction (44).

25. Implant selon l'une des revendications 21 à 24, **caractérisé en ce que** la surface d'appui de corps de vertèbre (46) est située sur la face opposée où se trouve ledit au moins un logement d'organe de maintien d'élément de fixation (52).

26. Implant selon l'une des revendications précédentes, **caractérisé en ce que** ledit au moins un organe de maintien d'élément de fixation (24, 26) présente au moins un élément de réception d'outil (78, 98) pour un instrument de maintien et/ou de mise en place.

27. Implant selon l'une des revendications précédentes, **caractérisé par** au moins deux éléments de fixation (28) pouvant être introduits au moins partiellement dans un corps de vertèbre (12, 14).

28. Implant selon la revendication 27, **caractérisé en ce que** l'un au moins des organes de maintien d'élément de fixation (24, 26) est, dans une position de mise en place, monté de manière mobile sur l'un des éléments de fixation (28), et **en ce que** dans une position de liaison, ledit au moins un organe de maintien d'élément de fixation (24, 26) est fixé de manière non mobile à cet élément de fixation (28).

29. Implant selon la revendication 27 ou la revendication 28, **caractérisé en ce que** l'un au moins des éléments de fixation (28) est réalisé sous la forme d'une vis à os ou vis d'ostéosynthèse (28).

30. Implant selon la revendication 29, **caractérisé en ce que** la vis d'ostéosynthèse (28) est réalisée sous la forme d'une vis polyaxiale (28) qui comprend une tête d'articulation (80), et **en ce que** l'un au moins des organes de maintien d'élément de fixation (24, 26) présente un logement de tête d'articulation (66, 94) correspondant à la tête d'articulation (80) et destiné à cette tête d'articulation (80).

31. Implant selon l'une des revendications 27 à 30, **caractérisé en ce que** l'un au moins des éléments de fixation (28) peut être fixé à l'aide d'un élément d'arrêt ou de sécurité au dit au moins un organe de maintien d'élément de fixation (24, 26).

32. Implant selon l'une des revendications 27 à 31, **caractérisé en ce que** le système d'implant (10) englobe un système d'instrument pour la mise en place des éléments de fixation (28) dans un corps de vertèbre (12, 14) et/ou pour fixer au moins un élément de fixation (28) à au moins un organe de maintien d'élément de fixation (24, 26).
